# EUROPEAN PATENT APPLICATION

(11) **EP 4 621 797 A1**
(43) Date of publication of application: **24.09.2025**
(21) Application number: 25165130.3
(22) Date of filing: 20.03.2025
(51) Int. Cl.: G16H 40/40, G16H 40/67, A61N 1/372

(54) **SYSTEM AND METHOD FOR IMPLANTABLE MEDICAL DEVICE REMOTE PROGRAMMING**

(30) Priority: 22.03.2024 US 202463569010 P; 19.03.2025 US 202519084382
(71) Applicant: Pacesetter, Inc., Sylmar, CA 91342 (US)
(72) Inventor: Glasser, Steven, Sylmar, 91342 (US); Iyer, Ashok, Sylmar, 91342 (US); Singh, Tejpal, Sylmar, 91342 (US); Deshmukh, Nilesh, Sylmar, 91342 (US); Malhotra, Neha, Sylmar, 91342 (US)
(74) Representative: Barker Brettell Sweden AB

(57) **Abstract**

A communication system (100, 300) is provided that includes a remote electronic device (116, 304) configured to communicate with a medical device (114, 212, 302) of a patient via a local programming electronic device (106, 306). The remote electronic device (116, 304) can include one or more processors (202, 316) configured to control operations of the local programming electronic device (106, 306) to program the medical device (114, 212, 302) during a dynamic session. The one or more processors (202, 316) can also be configured to terminate the dynamic session in response to 1) a persistent action of a user (112) of the remote electronic device (116, 304) exceeding a static persistent state threshold and 2) a monitored event exceeding a dynamic threshold.

## Description

### TECHNICAL FIELD

Embodiments of the present disclosure relate to methods, systems, and devices for updating operating configurations of medical devices via remote communications and providing medical care from a remote location.

### BACKGROUND

Medical devices can be utilized to monitor, treat, etc. patients in a variety of ways. Some medical devices can be external to the patient, such as a heart monitor, heart assist devices, neuromodulation devices, blood glucose monitors, ketone monitors, lactate and alcohol monitors, heart pumps, infusion pumps, deep brain stims, smart wearable devices such as smart watches, rings, patches, etc. that can monitor health related characteristics of a patient. Other medical devices are implanted in the patient.

An implantable medical device (IMD) is a medical device that is configured to be implanted within a patient anatomy and commonly employs one or more electrodes that either receive or deliver voltage, current or other electromagnetic pulses from or to an organ or tissue for diagnostic or therapeutic purposes. In general, IMDs can include a battery, electronic circuitry, a pulse generator, a transceiver, a microprocessor, or the like. The microprocessor is configured to manage communication with an external device or instrument as well as control patient therapy. The components of the IMD are hermetically sealed within a housing. The IMD is completely enclosed within the human body. Thus, there is no means of direct interaction between an IMD and a remote electronic device, other than through wireless communication.

As technologies advance, new ways of providing medical care for patients, including those with IMDs, are provided. With communications advances, remote health care by a clinician of a patient is becoming more common, with patients communicating with a clinician via web conferencing. In addition, remote communication, and programming of patient medical devices can also occur remotely.

For example, an IMD may require programming updates over time to adjust the behavior and operations of the IMD. The programming updates are wirelessly communicated from a local programming electronic device outside of the patient to the IMD within the patient. When updating the IMD, care must be taken to ensure that the software, firmware, application, program, or the like that is being updated is the software, firmware, application, program, or the like of the update. For example, when a patient experiences a health issue with their IMD and goes to a hospital, a clinician at the hospital can change a treatment, updates the software, firmware, application, program, or the like of the IMD. If the patient then goes to their regular clinician, and the regular clinician has a different treatment, or update they desire to implement, it is important to the regular clinician to know the current treatment, updates to the software, firmware, application, program, etc. before making such a change. Otherwise, mistakes can occur related to the update, potentially harming the patient.

Still, a need for greater remote control is still desired. By having remote control over an IMD or other medical device local to the patient, burden can be removed from field teams. Field representatives can reduce travel and expense by using remote communication and programming, freeing up these field representatives for more complex cases. Field representatives can include anyone with the appropriate knowledge and training to perform programming of a medical device. Such field representatives can include clinicians, including at a location of a medical device, or remote, vendor-technical support, or the like. In addition, by having remote control of health care, including IMDs, necessary care can be provided in a timely manner without the patient having to wait for a field representative to show up at the location of the patient.

In addition, greater demand is present for remote medical care. Clinicians and clinician support representatives desire to communicate with one another to diagnosis conditions, provide treatments, and the like for a local patient while the clinician support representative is at a remote location.

When using remote medical care, a remote user may read or modify the state of a patient's medical device or interact with any external system or device connected to a programmer while supporting a local user (e.g., nurse, device technician, physician, or other individual adequately trained to use and support such systems). In some cases, the local user training may limit what such users can, or are willing to, perform using the programmer. Therefore, remote control systems benefit from being capable of independent operation with minimum support from the local user. This allows the patient to receive the best care possible during their clinical encounter. In this method and system, local users can always take immediate control at any time to ensure the safe operation of the system if the patient's condition changes. Certain functions may also be prohibited for remote users, if the safety of performing such functions remotely is in question.

In all, a need remains for improved methods, devices and systems for administering remote communications, including programming communications to update a medical device.

### SUMMARY

In accordance with an embodiment, a communication system is provided that includes a remote electronic device configured to communicate with a medical device of a patient via a local programming electronic device. The remote electronic device can include one or more processors configured to control operations of the local programming electronic device to program the medical device during a dynamic session. The one or more processors can also be configured to terminate the dynamic session in response to 1) a persistent action of a user of the remote electronic device exceeding a static persistent state threshold and 2) a monitored event exceeding a dynamic threshold.

Optionally, the one or more processors can be further configured to provide a timer during the dynamic session and determine whether a duration of the persistent action of as determined by the timer exceeds the static persistent state threshold.

In one example, the one or more processors may be further configured to adjust the timer based on a manual input from the user of the remote electronic device.

In one example, the one or more processors can be further configured to obtain patient characteristics, clinician characteristics, medical device characteristics, or monitoring characteristics, determine the static persistent state threshold based on the patient characteristics, the clinician characteristics, the medical device characteristics, or the monitoring characteristics obtained, and update the static persistent state threshold based on the determining.

Optionally, the one or more processors can be further configured to obtain patient characteristics, medical device characteristics, or monitoring characteristics, and determine the dynamic threshold based on the patient characteristics, the medical device characteristics, or the monitoring characteristics obtained.

In one example, the monitoring characteristic can be related to connectivity between the remote electronic device and the local programming electronic device.

In one example, the dynamic session is a programming session.

In one example, the static persistent state threshold may be less than five seconds.

In one example, the one or more processors can be further configured to revert the medical device to a previous configuration via the local programming electronic device in response to termination of the dynamic session.

In accordance with an embodiment, a communication system is provided that can include a remote electronic device including one or more processors configured to communicate with a local programming electronic device. The one or more processors of the remote electronic device can also be configured to control the local programming electronic device to program a medical device of a patient. The local programming electronic device may include one or more processors configured to program the medical device during a dynamic session, monitor persistent actions of the remote electronic device during the dynamic session, and monitor at least one of patient characteristics, medical device characteristics, or monitoring characteristics during the dynamic session. The one or more processors of the local programming electronic device can also be configured to determine a dynamic threshold based on the patient characteristics, the medical device characteristics, or the monitoring characteristics, determine whether the dynamic threshold is exceeded during the dynamic session, and determine whether a persistent action of the persistent actions exceeds a static persistent state threshold during the dynamic session. The one or more processors of the local programming electronic device can further be configured to terminate the dynamic session when either 1) the dynamic threshold is exceeded, or 2) when the persistent action exceeds the static persistent state threshold.

In an example, the one or more processors of the remote electronic device is configured to terminate the dynamic session in response to the persistent action of the user of the remote electronic device exceeding the static persistent state threshold. In another example, the one or more processors of the remote electronic device is configured to terminate the dynamic session in response to the monitored event exceeding the dynamic threshold. In a further example, the one or more processors of the remote electronic device is configured to terminate the dynamic session in response to the persistent action of the user of the remote electronic device exceeding the static persistent state threshold and the monitored event exceeding the dynamic threshold.

Optionally, the one or more processors of the local programming electronic device can be further configured to provide a timer during the dynamic session and determine whether a duration of the persistent action as determined by the timer exceeds the static persistent state threshold.

In one example, the one or more processors of the remote electronic device can be further configured to adjust the timer based on a manual input from the user of the remote electronic device.

In one example, the one or more processors of the local programming electronic device can be further configured to determine the static persistent state threshold based on clinician characteristics, the patient characteristics, the medical device characteristics, or the monitoring characteristics obtained, and update the static persistent state threshold based on the determining.

In one example, the monitoring characteristic may be related to connectivity between the remote electronic device and the local programming electronic device.

In one example, the persistent action can include pressing an input button.

In one example, the dynamic session can be a programming session.

In one example the one or more processors of the local programming electronic device may be further configured to revert the medical device to a previous configuration in response to termination of the dynamic session so that the medical device can provide a treatment to the patient.

In accordance with an embodiment, a communication method is provided that can include controlling a local programming electronic device with a remote electronic device to program a medical device of a patient during a dynamic session and monitoring persistent actions of the remote electronic device during the dynamic session. The method can also include determining whether a persistent action of the persistent actions exceeds a static persistent state threshold during the dynamic session, terminating the dynamic session when the persistent action exceeds the static persistent state threshold, and reverting the medical device to a previous configuration in response to terminating the dynamic session to treat the patient.

Optionally, the method can also include monitoring at least one of patient characteristics, medical device characteristics, or monitoring characteristics during the dynamic session and determining a dynamic threshold based on the patient characteristics, the medical device characteristics, or the monitoring characteristics. The method may also include determining whether the dynamic threshold is exceeded during the dynamic session, and terminating the dynamic session when the dynamic threshold is exceeded.

In one example, determining the static persistent state threshold can include at least one of 1) receiving a manual input from a remote user of the remote electronic device or 2) determining the static persistent state threshold based on patient characteristics, clinician characteristics, medical device characteristics, or monitoring characteristics.

### BRIEF DESCRIPTION OF THE DRAWINGS

Figure 1 is a schematic block flow diagram of a system operated, according to an embodiment.
Figure 2 is a schematic block diagram of an electronic device, according to an embodiment.
Figure 3 is a schematic illustration of a system, according to an embodiment.
Figure 4 is a simplified block diagram of a system operated in accordance with embodiments herein.
Figure 5 is a block diagram of medical device, according to an embodiment.
Figure 6 is a flow block diagram of a method for providing inputs to an IMD from a remote electronic device, according to an embodiment.
Figure 7 is a schematic diagram of an input screen utilized during a device session, according to an embodiment.
Figure 8 is a schematic diagram of an input screen utilized during a device session, according to an embodiment.

### DETAILED DESCRIPTION

Embodiments herein provide methods, devices and communication systems that are designed to enhance integrity and authenticity of wireless programming communications between one or more remote electronic devices and a medical device, such as an IMD. The embodiments assist in the safe termination of temporary actions due to persistent actions of a remote user exceeding a static persistent state threshold, or because of a dynamically monitored event.

During a communication session between a remote electronic device used by a remote user at a remote location and a local programming electronic device that is local to a medical device of a patient and used to program the medical device (also referred to as a remote session) a dynamic session can occur where the remote electronic device controls the local programming electronic device to program a local medical device, such as an IMD. During this dynamic session, numerous modes of remote user input can be provided. Modes of user input to the system are varied and include, but are not limited to, pressing buttons or sliding action widgets, touch screen controls or virtual reality peripherals, hand or motion gestures (detected optically, electrically, or otherwise), voice commands and/or sounds, facial recognition or any similar form of human-to-device interaction. Some user inputs may be singular and instantaneous events while others may involve a series of actions over an extended period of time. An example of an extended action includes the remote user pressing and holding a console touch screen (or holding a mouse button in a depressed state) to start an action, then releasing the touch screen (or mouse button) at a later time to end that action. User inputs which are not instantaneous can therefore be used to control sequential actions or those actions where the duration is expected to be user controlled and limited in time such as in the example provided above. These extended actions with a defined beginning and end based on user input are referred herein as persistent actions.

The capabilities of the remote user to initiate persistent events may take many forms. Persistent events may include changing the device state temporarily, or beginning a sequence of events (i.e., tests) where the duration of such events is variable in nature and should persist only as long as the remote user indicates and/or while the state of the various interacting systems is satisfactory. One example of such events and conditions is a manual decrement capture test for an implantable cardiac rhythm monitoring device, where a loss of pacing could result in failure to deliver the necessary pacing therapy leading to inadequate cardiac output, if the temporary state of the system persists after pacing capture is lost or the patients' condition otherwise changes for any reason. The inputs by the operators of the local programming electronic device (e.g., either a local user and/or remote user) are essential to ensure temporary conditions are not persisted longer than necessary. The system provided terminates persistent actions, which may or may not be associated with temporary changes to the operating condition of the system by the local or remote user, upon any state change monitored by the system, which includes, but is not limited to, the conditions (state) of the components in the system, the patient's physical condition, or the communication channel between any component of the system (i.e. the remote electronic device and the local programming electronic device).

As stated above, there are two means of safely terminating persistent actions, which can be used independently or together as necessary: static thresholds and dynamic monitoring. Static thresholds terminate any persistent actions after a pre-programmed time limit, or count of events, as managed independently within the medical device, or within the local programming electronic device used to program the medical device. Termination of an action performed by the remote electronic device due to a loss of communication with the remote electronic device may be limited to actions, which were initiated by a remote user. Static thresholds could be fixed or configurable to extend the utility of the local programming electronic device such that the thresholds may adapt to the different needs and applications for which it is used. For example, the timing required to safely self-terminate one action may vary from action-to-action or patient-to-patient, and so on. A remote user action button, or similar interface, can also be provided to configure the system such that the fixed threshold for subsequent persistent actions can vary. Such designs can account for the level of safety required for a given action, the level of training of the remote user or the local user, the patient's physiology or any other factor.

The local programming electronic device uses the static threshold default value, or the user provided threshold, to force the termination of persistent events independently. The event is terminated based on a locally implemented counter or timer after the action was initiated (such as by the remote user), regardless of the state of the system (or the state of connectivity between the systems or any other property). The advantage of this design is that it provides independent safety controls with flexibility. The flexibility is provided to address utility since some persistent actions are not associated with safety concerns, or where the safety of the system can tolerate longer breaks in communication or allows for more variation in the condition of the system or patient. Whereas shorter durations are used by default and wherever appropriate. By implementing these controls in an independent layer of the system, such as between the remote electronic device and the local programming electronic device and which communicate with the other connected devices and systems, this safety mechanism works independently and would apply to any aspect of the operation of the local programming electronic device.

A challenge of the static (with or without configuration) designs is that a fixed short threshold, necessary for more critical features or where the training of the local or remote user cannot be ensured, may not be adequate to complete the broader set of tasks supported by the local programming electronic device where persistent user input is also used to control the system. In this context, persistent implies a singular but extended action duration, such as the simple case of pressing and holding a button without releasing the button. Variations of design, such as allowing configurable thresholds, are possible but add complexity and the potential for use errors. Features to re-initialize or revert the threshold to its safest value become necessary and have to address a wide range of factors which are more difficult to use and maintain. For these reasons, terminating the persistent action based on dynamic monitoring of the system state is also considered.

Dynamic monitoring may be used independently or to extend the utility of a static persistent state threshold. Dynamic monitoring can end the persistent (local or remote user) action only upon detecting a change in the state of the system (such as due to the loss of communication between the remote electronic device and the local programming electronic device, a change in state of the local programming electronic device or of a connected system such as a medical device like an IMD, a user input from the local user, a change in state or condition of a patient or the patient's implantable device as detected through any channel such as an external heart monitor or alarm, etc.) If the change in state cannot be detected directly, and instantaneously, use of a fixed static persistent state threshold can be used in addition to the periodic monitoring of the dynamic input(s). For example, the beginning of the static time evaluation can be reset periodically, such as each time the dynamic status is successfully re-evaluated. Thus, the persistent action is automatically terminated only after the combination of reaching the pre-defined static persistent state threshold since the last dynamic status event was re-evaluated. Whereas some dynamic status events may be independently monitored completely within the local programming electronic device, other status events may involve the periodic transfer of information between systems. For example, the remote electronic device (or other externally monitored source of input) may periodically (such as once every 100 ms, once per second, once per minute, etc.) send a status update to the local programming electronic device to confirm communication between the two devices is present, that communication latencies are adequate for such actions, or that some other criteria essential to safety or performance of the system remains satisfied. If the static persistent state threshold (i.e. a timer expires), described above, is reached after the most recent periodic status update is received by the local programming electronic device, this could be interpreted as a loss of communication necessitating the automatic end of the persistent action, since remote user input cannot be transmitted to the local programming electronic device when that communication channel is not available.

It will be readily understood that the components of the embodiments as generally described and illustrated in the figures herein and may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems, devices and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

The term "local programming electronic device," as used herein refers to any and all electronic devices utilized by a nurse, caretaker, device technician, physician, other individual adequately trained to use and support such systems, or the like that is local, or in near proximity to the patient, and is used to program a medical device related to the patient. The local programming electronic device can be a central processing unit (CPU), desktop computer, laptop computer, smartphone, smart watch, monitor, handheld device, portable or industrial devices, such as activators, tablets, consoles including monitor consoles, or the like which interact with medical devices to retrieve or update any information or settings during a communication session with a remote user. In one example, the local programming device can be within an IMD of the patient. To this end, an IMD that includes the functionality of a local programming electronic device as described herein is considered to be an IMD with a local programming electronic device whether a physically separate device is provided from the IMD or not. The local programming electronic device includes one or more processors configured to follow instructions, and a transceiver configured to communicate over a network, in a cloud, over Bluetooth (BLE), wirelessly, over the air, through a wire, or the like. In one example, the local programming electronic device communicates with a patient device. In another example, the local programming electronic device is local to the patient and can communicate with a medical device, including an IMD of a patient. In this manner, the local programming electronic device can be located locally at the patient and communicate with a medical device of the patient. In addition, while the term "programming" is utilized as part of the term "local programming electronic device" a local electronic device that does program, such as a local electronic device that causes a dynamic occurrence such as an interrogation of the medical device can be considered a local programming electronic device.

The term "remote electronic device," as used herein refers to any and all electronic devices that are not located near the patient. The remote electronic device must communicate with an electronic device, such as a programming device, or other type of local programming electronic device or the like at the location of the patient over a network, mesh network, wirelessly, wired, or the like. In one example, the remote electronic device communicates with a local programming electronic device that can be local to a patient, and in communication with the remote electronic device. In an example, the local programming electronic device is configured to be operated by a nurse, caretaker, device technician, physician, other individual adequately trained to use and support such systems, or the like, whereas the remote electronic device can be operated by a clinician, a clinician support representative, etc. In particular, the remote electronic device communicates with the local programming electronic device via a network, engine, etc.

The term "communication session" as used herein is any period of time dedicated to communication between a remote electronic device and a local programming electronic device over a network, mesh network, wirelessly, wired, etc. to provide information and data between a patient and medical personnel (e.g., clinician) or between two or more medical personnel (e.g., clinician and clinician support representative). In an example, the communication session between the local programming electronic device and remote electronic device can conducted via a network, an engine within the network at cloud location, etc.

The term "device session" as used herein is any period of time when a local programming electronic device is in communication with a medical device that is local to the local programming electronic device. In one example, the medical device can be an IMD. The device session can and often occurs during the communication session between the remote electronic device and the local programming electronic device. In such examples, the remote electronic device can control the local programming electronic device to communicate with and program the medical device.

The term "dynamic session" as used herein is a period of time when a medical device is in communication with another electronic device and changes are made to the medical device during the period of time. In examples, the changes include updates, modifications, alterations, interrogations, or the like that are made to either software or hardware of the medical device. In examples the changes can include those made to parameters used by the medical device to function for the intended purpose of the medical device. For example, a parameter can be a threshold used to determine whether to indicate a diagnosis, provide pacing or other treatment, or the like. In examples the electronic device can be a local programming electronic device, remote electronic device that communicates with the medical device via the local programming electronic device, or the like. In an example, the dynamic session can occur during a communication session and device session. In one example, the dynamic session is a period of time that an electronic device, such as a local programming electronic device and/or remote electronic device, operates to program the medical device. In an example, the dynamic session occurs during a device session when the local programming electronic device communicates with the medical device. When the local programming electronic device is in a communication session with the remote electronic device the remote electronic device can provide inputs for the medical device via the local programming electronic device during the dynamic session. In another example, during the dynamic session an electronic device, such as a local programming electronic device and/or remote electronic device, operates to interrogate the medical device to obtain medical device data and information, to ensure proper operation of the medical device, or the like.

The term "to program" as used herein refers to any and all updates, changes, modifications, alterations, or the like made directly to software or hardware. In example embodiments, this "programming" is implemented by a remote electronic device to a medical device or local programming electronic device or indirectly where the remote user updates the settings or configuration of the medical device or local programming electronic device via the connection from the remote electronic device. For example, there are some device tests that are run as a group. The selection of what tests to run and their configuration is controlled by the local programming electronic device (programmer) software. The local user can set all the parameters to be used for all the tests in question and then run them in a batch/group. While the local user does not participate in some persistent actions, such as holding a mouse button down throughout such test flows, similar concepts apply. In addition, a design can be provided where the remote user can now set all the same test parameters remotely. If the remote electronic device loses remote connectivity, or a change in patient condition requires, aborting the entire series of tests would be appropriate. In yet another embodiment, is a case where "resetting" the state of the IMD and the programmer would be appropriate to terminate the batched series of tasks.

The terms "persistent events" and "persistent actions" as used interchangeably herein refers to an event or action that continues to exist or endure over a prolonged period of time. Examples of persistent events and persistent actions include any human to machine interactions, inputs, physical pressing, compressing, etc. performed by a user of an electronic device during a dynamic session. In an example, the persistent event or action is the pressing of an input button on a keyboard of an electronic device during the dynamic session. In another example, touching an input on a touch sensitive input screen is a persistent event or action during a dynamic session. In another example, an auditory instruction that can be implemented by an electronic device is a persistent event or action. In each example, the action occurs over a prolonged or extended period of time. To this end, an extended action (e.g., persistent action) can also include a remote user pressing and holding a console touch screen (or holding a mouse button in a depressed state) to start an action, then releasing the touch screen (or mouse button) at a later time to end that action. In one example, the prolonged period of time can be any time that is equal to or greater than two seconds.

The term "static persistent state threshold" as used herein indicates a determined amount of time, in which a persistent event or persistent action occurs. In one example, the static persistent state may be measured in seconds, while in other examples may be measured in milliseconds, minutes, other time increment, etc. To this end the static persistent state threshold may be 2 seconds, 3.5 second, 5 seconds, 10.932 seconds, or the like. It may also be configured to be indefinite in duration, in effect bypassing this functionality if appropriate. A static persistent state threshold may be determined by an electronic device based on characteristics, input into an electronic device manually, or the like. Still, once a dynamic session begins, the static persistent state threshold can only be changed or updated by a manual input during the dynamic session, such as if the user cancels the programming operation.

The term "monitored event" as used herein refers to any occurrence of a process that is detected by a sensor. In examples, the process can be an amount of time reaching a threshold, a sensed voltage reaching a threshold, a detected oxygen level reaching a threshold, a determined amount of time during a determined period of time, or the like. In a particular example, the monitored event represents an amount of time for a monitored parameter reaching a threshold. Examples of sensors can be any sensor of an electronic device that detects the functioning of the electronic device. Examples of sensors may also be physiological sensors that are configured to sense respiration rate, pH of blood, ventricular gradient, activity, body movement, position/posture, minute ventilation (MV), and/or the like. In an example, the monitored event can be the amount of time a key on a keyboard of an electronic device is compressed.

The term "dynamic threshold" as used herein refers to a threshold, such as a limit, maximum, minimum, baseline, etc. that is continuously and repeatedly determined, calculated, obtained, in real time based on characteristics including data and information that is continuously and repeatedly obtained in real time. The characteristics can include patient characteristics, clinician characteristics, monitoring characteristics, medical device characteristics, IMD characteristics, or the like. The dynamic threshold can be measured in time, oxygen level or volume, pressure, temperature, pulse rate, electrical value, voltage, current, rate or the like and can be measured, determined, calculated, obtained, etc.

The term "real time" as used herein shall mean at the same time, or a time substantially contemporaneous, with an occurrence of another event or action. For the avoidance of doubt, as an example, when a dynamic threshold is dynamically determined, changed, updated, etc. in real-time the dynamic threshold is determined, changed, updated, etc. each couple of milliseconds, each couple of seconds.

The term "monitored event" as used herein refers to any condition, status, measurement, determination, calculation, or the like related to the dynamic threshold. For example, when the dynamic threshold is based on a measured blood pressure of a patient, obtaining the blood pressure is the monitored event. In another example, when the dynamic threshold is a risk score associated with a health risk of a patient that is determined via calculation, modeling, decision tree, lookup table, etc., determining the risk score is the monitored event. To this end, the monitored event can be, but does not have to be, a direct measurement that is obtained and instead can be determined, calculated, etc. based on more than one factor, characteristic, parameter, or the like.

The term "obtains" and "obtaining", as used herein in connection with data, signals, information, and the like, include at least one of i) accessing memory of an electronic device including a remote server where the data, signals, information, etc. are stored, ii) receiving the data, signals, information, etc. over a wireless communications link between the medical device and a local programming electronic device, iii) receiving the data, signals, information, etc. over a wired or wireless communications link and transceiver between a sensor, peripheral, diagnostic device or other system connected to the local programming electronic device and/or iv) receiving the data, signals, information, etc. at a remote server over a network connection. The obtaining operation, when from the perspective of an IMD, may include sensing new signals in real time, and/or accessing memory to read stored data, signals, information, etc. from memory within the IMD. The obtaining operation, when from the perspective of a local programming electronic device, includes receiving the data, signals, information, etc. at a transceiver of the local programming electronic device where the data, signals, information, etc. are transmitted from a medical device, a diagnostic sensor or system and/or a remote server. The obtaining operation may be from the perspective of a remote server, such as when receiving the data, signals, information, etc. at a network interface from a local programming electronic device and/or directly from a medical device. The remote server may also obtain the data, signals, information, etc. from local memory and/or from other memory, such as within a cloud storage environment and/or from the memory of a workstation or clinician programming device.

The term "engine" as used herein refers to any processor, electronic device with a processor, server, or the like within a cloud, network, etc. configured to communicate with electronic devices via a network, mesh network, wirelessly, wired, or the like that can make determinations. In one example, the engine can be a remote programming engine (RPE) that communicates with a local programming electronic device. In another example, the RPE communicates between a local programming electronic device, and a remote electronic device.

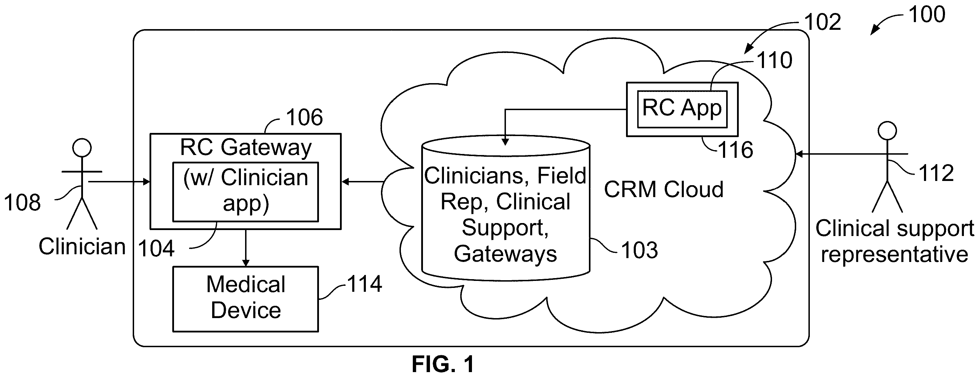

Figures 1-2 illustrate an embodiment of a communication system for providing remote medical care. In this embodiment, a medical device remote-control system (MDRC system) provides a secure, safe, end-to-end process for programming a medical device via a remote electronic device and a local programming device. All communications and operations between the remote user and the local user through the supported connectivity protocols (including Cloud network and Bluetooth) are securely signed and encrypted. To this end, optionally, all such communication sessions can be recorded, and all operations can be logged.

Figure 1 illustrates a MDRC system 100 that includes an engine 102, medical database 103, a programming application 104 that can be accessed within a local programming electronic device 106 for local users 108 (e.g., nurse, caretaker, device technician, physician, other individual adequately trained to use and support such systems, or the like), a remote control (RC) application 110 at a remote electronic device 116 for clinical support representatives 112 (e.g., clinician, clinical support representatives, or the like), and at least one medical device 114. By providing the MDRC system 100, communication sessions, device session, and dynamic session can all be provided.

The engine 102 can include one or more processors for executing instructions to perform processes and methods described herein. The engine 102 can also include a memory or storage device for storing information, including patient information, medical device information, treatment information, etc. In an example, the engine 102 is within a cloud environment that can be accessed by numerous electronic devices other than the local programming electronic device 106 and the remote electronic device 116. In one example, the engine 102 is coupled to a medical database 103 that includes medical device information, electronic device information, including electronic device information related to the local programming electronic device 106 and the remote electronic device 116, active or historical remote sessions, or the like. The information stored can be related to an individual patient, numerous patients, patient medical devices, generic medical devices, studies, tests, other patient information, etc. In all, the medical database 103 and engine 102 can be coupled within a network, including a mesh network, cloud network, Bluetooth network, etc. and communicate with plural local programming electronic devices, plural remote electronic devices, etc. to obtain information related to plural patients, medical devices, treatments, or the like. In this manner, the medical database 103 can include algorithms, including artificial intelligence algorithms for analyzing information obtained from the plural patients, medical devices, treatments, etc. to program, provide recommendations, modifications, updates, changes, or the like to medical devices, treatments, etc. accordingly.

The local programming electronic device 106 is the electronic device that is at location of the patient, and/or medical device 114. The local programming electronic device 106 is considered local because the patient, and/or medical device 114 is at the location of the local programming electronic device 106 and is not remote from the patient and/or medical device 114. In one example, the medical device 114 is an IMD. In another example, the medical device 114 may be an Insertable Cardiac Monitor (ICM), neurostimulation device, heart pump, sensor, clothing accessory, external patient monitor, or the like. As such, medical device 114 when used throughout this document refers to any such type of medical device or sensor regardless of whether that device is held by the patient, inserted, implanted or worn externally and temporarily. The local programming electronic device 106 can be an industrial medical device, laptop computer, desktop computer, mobile device, server, tablet (i.e. iPad), or the like.

The local programming electronic device 106 can include one or more processors for executing instructions to perform processes and methods described herein. The local programming electronic device 106 can also include a memory or storage device for storing information, including patient information, medical device information, treatment information, etc. The local programming electronic device 106 can also include an interface so that a local user 108, such as a nurse, caretaker, device technician, physician, other individual adequately trained to use and support such systems, or the like, can input information into the local programming electronic device 106. The interface in one example is an input device that can include a touch screen, keyboard, mouse, microphone, or the like to provide information, commands, etc. to the local programming electronic device 106. In addition, the local programming electronic device 106 can include an output device, such as a screen, display, speakers, etc. for providing information and data to the local user 108. The local programming electronic device 106 can thus be utilized by the local user 108 to obtain information directly from the medical device 114, and/or a patient that has the medical device 114.

When a local user requests and approves support from a remote user, upon powering up the local programming electronic device 106 as appropriate to the design embodiment, or the like, the local programming electronic device 106 detects network connectivity and automatically performs an analysis to ensure the engine 102 is operational and connected. The connection is qualified and determined by the identification, name, serial number, etc. associated with the local programming electronic device 106, the version of the software, firmware, or the like of the clinician application 104, operating system, etc. of the local programming electronic device 106, the location or region of the local programming electronic device 106, or the like. This information can be used to confirm compatibility of the system components, enhance the performance or resources used by the system, or to configure aspects of the system as necessary to meet local geographical and/or legal requirements.

The remote electronic device 116 is located remotely, or away from the patient and/or medical device 114. The remote electronic device 116 may typically be located in a physically distant hospital, clinician's office, clinician's home, or the like that is not in the location of the patient and/or medical device 114, but it may also be used nearby but not adjacent to the patient and/or medical device 114. The remote electronic device 116 can be a laptop computer, desktop computer, mobile device, server, tablet (i.e. iPad), or the like that includes an interface, such as a browser window or application, so that a clinical support representative 112, such as a clinician, clinician support representative, or the like, can input information into the remote electronic device 116. The remote electronic device 116 in one example is an electronic device of a network.

The remote electronic device 116 can include one or more processors and a memory for executing instructions and storing information like the local programming electronic device 106 and engine 102. In addition, the remote electronic device 116, like the local programming electronic device 106, can include an interface that is an input device, along with an output device. The remote electronic device 116 also includes the RC application 110 that includes program instructions for obtaining patient information from the medical database 103 via the engine 102. The RC application 110 can also communicate with the clinician application 104 to obtain physiological information related to a patient that is obtained from the medical device 114 via the engine 102. The user interface of the local programming electronic device 106 may be shared directly with the Clinical support representative 112 through the remote electronic device 116 and RC application 110 allowing the remote user to interact with the local programming electronic device 106 as if they were standing next to that device 106 and the patient.

When a clinical support representative 112 turns on the remote electronic device 116, the remote electronic device 116 authenticates and registers the user. Upon power on and/or approval of the local user to start a communication session that is considered a remote session using the local programming electronic device 106, the remote electronic device 116 automatically performs an analysis to ensure the engine 102 is operational with the local programming electronic device 106 based on the identification, name, serial number, etc. associated with the local programming electronic device 106, the version of the software, firmware, or the like of the RC application 110, operating system, etc. of the remote electronic device 116, the location or region of the remote electronic device 116, or the like. Alternatively, in other embodiments, the local user initiates the remote session.

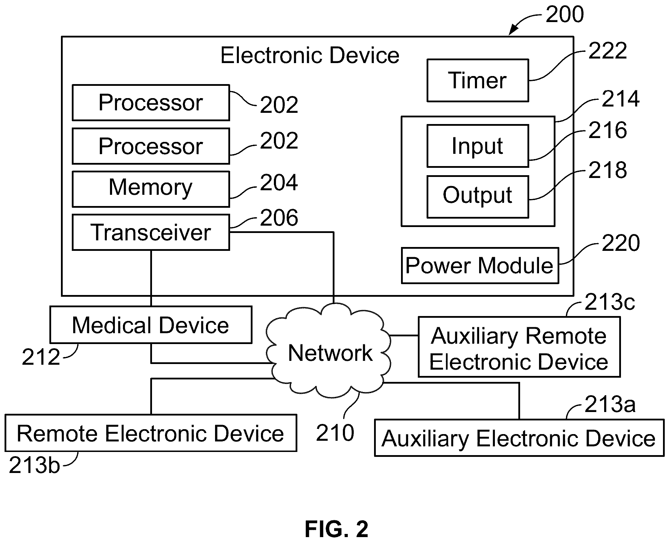

Fig. 2 illustrates a schematic block diagram of an electronic device 200. In one example, the electronic device 200 is the local programming electronic device 106 of Fig. 1, whereas in another example the electronic device 200 can be the remote electronic device 116 of Fig. 1. The electronic device 200 can be an industrial medical device, laptop computer, desktop computer, mobile device, server, tablet (i.e. iPad), or the like.

The electronic device 200 can include one or more processors 202, a storage device or memory 204, and a transceiver 206. The transceiver 206 is configured to communicate with other electronic devices through a network 210. To this end, the transceiver can communicate with a server, engine, or the like, located in the cloud, over Bluetooth, etc. In addition, the transceiver can communicate with a medical device 212, such as the medical device 114 in Fig. 1.

Medical device 212 can be any device similar to that described herein as an IMD or medical device of any of the Figures herein. The transceiver 206 can communicate with the medical device 212 remotely through the network 210, or directly. In another example, the transceiver 206 can communicate with an auxiliary electronic device 213a, a remote electronic device 213b, or a remote auxiliary electronic device 213c. In example embodiments, the auxiliary electronic device 213a can be a smartphone, tablet (i.e. iPad^{®}), or the like that can receive text messages, such as short message service (SMS) messages over the network. The remote auxiliary electronic device 213c similarly can be a smartphone, tablet (i.e. iPad^{®}), or the like that can also receive text or SMS messages. In this manner, the electronic device 200 can receive identification information from a clinician, clinician support representative, etc. and a text or SMS message can be provided by the electronic device 200 to the auxiliary electronic device 213a and/or 213c to provide verification that the clinician, or clinician support representative indeed provided the identification information. The identification information can be a username, password, login, or the like. By using a communication signal between the auxiliary electronic device 213a and remote auxiliary device 213c, additional security is provided. Text messages, such as short message service (SMS) messages, noted above could be replaced with any secure and encrypted form of communication which supports authentication and authorization of users and information exchanged over the network 210. This can include additional layers to meet the security demands of a system that includes the electronic devices described herein (e.g., electronic device 200 and auxiliary or remote electronic devices 213a-c), including but not limited to; two factor authentication, verbal challenges and authentication, non-repudiation checks, integrity checks and any additional functionality to ensure confidentiality, availability and integrity of the communication.

In one example, the electronic device 200, or any of the auxiliary or remote electronic devices 213a, 213b, 213c can provide a communication channel between a remote user and the medical device 212, such as an IMD. This communication channel can be directly to the medical device 212, or alternatively, can be provided indirectly as a result of the communication being made via a local programming electronic device, such as local programming device 106 in Fig. 1. Regardless of whether the communication channel is a direct connection or indirect connection, such electronic devices 200, 213a, 213b, 213c can each be utilized to provide a remote user with the communication path to the medical device 212 such that the remote user can have control over a dynamic session as described herein.

The electronic device 200 can also include an interface 214 that can include an input 216, such as a touchscreen, keyboard, mouse, microphone, etc. To this end, the input 216 can include buttons of a keyboard or mouse that can be pressed momentarily or held for some duration of time. The input 216 may also include sliding action widgets, touch screen controls or virtual reality peripherals. In another example, the input 216 can be a device that can detect hand or motion gestures (detected optically, electrically, or otherwise), voice commands and/or sounds, facial recognition, any similar form of human-to-device interaction, or the like. Thus, the input can be a camera, microphone, and/or other sensor for receiving data and/or information and/or commands and/or instructions from a user.

The electronic device 200 can also include an output 218 that can also include a touchscreen, display, speakers, etc. To this end, the input 216 and output 218 can include the same interface, display, etc. The electronic device 200 also includes a power module 220 for powering the electronic device 200. The electronic device 200 can also include a timer 222 that can be utilized to determine whether a persistent action has exceeded a persistent state threshold during a time the electronic device 200 is being utilized to program, update, change, or the like an IMD.

Persistent actions are actions, in which an activity is prolonged or endured, such as when a button, touch screen, etc. is actuated (e.g., held) for a period of time before a release, or ending of the actuation. The period of time can be a second, two seconds, five seconds, or the like. Alternatively, the action can be extended by repeatedly providing user input in some form such as pressing a user input multiple times during an interval, or period of time. Continued hand gestures, sound input, mechanical input, or the like may also be used to extend a user input action. For example, a button may need to be pressed three or more times within a five second interval. Thus, any form of user input which is repeated, such as pressing a button repeatedly during an interval of time, can also be considered a persistent action or event.

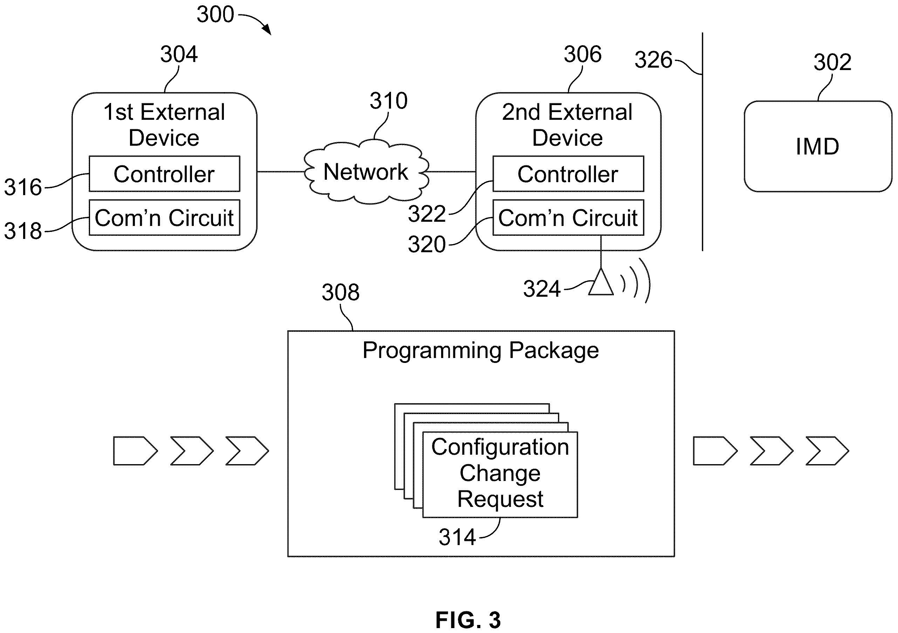

Figure 3 is a schematic illustration of a communication system 300 according to an embodiment. In one example, the communication system 300 is the system of Figure 1. The communication system 300 provides secure communication to a medical device, represented by an IMD 302 in the Figure 3. In addition to the IMD 302, the communication system 300 includes one or more external devices. In the illustrated embodiment, the external devices include a remote electronic device 304 and a local programming electronic device 306, but the communication system 300 may include more than two external devices in other embodiments. The remote electronic device 304 may be the remote electronic device 116 in Figure 1 and may be implemented as shown in Figure 2, and the local programming electronic device 306 may be the local programming electronic device 106 in Figure and may be implemented as shown in Figure 2.

In one example, the remote electronic device 304 can be remote from an IMD 302 and communicate with the local programming electronic device 306 to program the IMD 302. In such an embodiment, a remote user, such as a clinician, can control operation of the local programming electronic device 306 with the remote electronic device 304, including obtaining access to all information and functionality of the local programming electronic device 306. To this end, the remote electronic device 304 can control operation of the local programming electronic device 306 to provide user inputs, such as programming inputs, for the IMD 302. In such an embodiment, the local programming electronic device 306 may be local to the IMD 302 and operated and/or monitored by a nurse, caretaker, device technician, physician, other individual adequately trained to use and support such systems, or the like. The local user thus supervises the patient during such updating of the programming of the IMD 302. The local user may periodically assess the functionality of the communication system 300 to identify potential disruptions in communication, which may interfere with the ability of the remote electronic device 304 to control the local programming electronic device 306.

In this embodiment, the modes of user input by the remote electronic device 304 to the IMD 302 can be varied and include but are not limited to: pressing buttons or sliding action widgets, touch screen controls or virtual reality peripherals, hand or motion gestures (detected optically, electrically, or otherwise), voice commands and/or sounds, facial recognition, any similar form of human-to-device interaction, or the like. Some user inputs may be singular and instantaneous (momentary) events while others may involve a series of actions over an extended period of time. An example of an extended action includes a remote user pressing and holding a console touch screen (or holding a mouse button in a depressed state) to start an action, then releasing the touch screen (or mouse button) at a later time to end that action. User inputs which are not instantaneous can therefore be used to control sequential actions or those actions where the duration is expected to be user controlled and limited in time such as in the example provided above.

The capabilities of the remote user at the remote electronic device 304 to initiate persistent events with a local programming electronic device 306 that functions as a programming device may take many forms. Persistent events may include changing the device state temporarily, or beginning a sequence of events (i.e., tests) where the duration of such events is variable in nature and should persist only as long as the remote user indicates and/or while the state of the various interacting systems is satisfactory. For example, a manual decrement capture test for an implantable cardiac rhythm monitoring device can be provided, where continuing the test beyond the initial loss of capture results in a loss of pacing therapy. Continuing such a test beyond detection of the loss of capture, or the patients' condition or otherwise changes for any reason, may result in a failure to deliver the necessary pacing therapy to the patient leading to inadequate cardiac output. As a result, the communication system 300 allows the user to input a static persistent state threshold to limit how long the user has to complete the test before reverting the IMD 302 back to its previous state to ensure IMD 302 functionality is not compromised if the threshold is exceeded. So, if connection is lost resulting in the test not being completed before the static persistent state threshold, by having the static persistent state threshold the IMD 302 automatically reverts back to a previous state to prevent negative effects from an untimely test. This improves upon the manual monitoring of the communication system 300 described above and eliminates the need for manual intervention to ensure safety.

In another example, a portion of a test, programming, etc. may be to reset a function. To reset the function, a user input such as a key, space bar, return button, mouse, touch screen location, etc. must be compressed and held for an extended period, such as five seconds to cause the reset. The static persistent state threshold can then be set for 10 seconds for this action. In this manner, if a disconnection or other event occurs resulting in the compressed or held state to be considered continuing to occur past the 10 second static persistent state threshold, the IMD 302 functionality is automatically reverted accordingly.

In addition to the static persistent state threshold a dynamic threshold can be determined based on IMD characteristics, patient characteristics, monitoring characteristics, or the like where each determination is considered a monitored event. Patient characteristics include any and all data and information related to the patient. Example patient characteristics include patient age, health conditions, number of surgeries, medication being taken, current pulse rate, current blood pressure, or the like. The patient characteristics may be obtained from a memory of the remote electronic device 304, the local programming electronic device 306, a cloud-based device, another electronic device, input into the remote electronic device 304 or local programming electronic device 306, received from a local sensor, or the like. Clinician characteristics can include any and all data and information related to the clinician and/or their management of a patient(s). Information can include manual input provided by a clinician related to preferences of the clinician related to how long actions, tests, or the duration of programming should persist. Clinician characteristics can also be determined, calculated, etc. based on historical data related to other clinicians, physicians, standards of care, etc. that are performing the same or similar persistent actions. In one example the clinician may have a profile that includes the clinician characteristics. IMD characteristics includes all information and data related to the IMD 302. This can include information that can be input or obtained from a memory such as IMD type, model, age, usage, programming, or the like. IMD characteristics can also include data and information obtained from sensors related to the current operation of the IMD 302. Meanwhile, monitoring characteristics can include any and all information that can be obtained by a sensor of the communication system 300 or obtained by the communication system 300. This includes connectivity data, communication signal strength, environmental electrical noise or the like related to the communication between the remote electronic device 304 and the local programming electronic device 306. In addition, the monitoring characteristics can include information and data related to heart signals, pulse rate, oxygen levels, or the like that can be monitored. To this end, some patient characteristics may also be considered monitoring characteristics.

These characteristics may be monitored such that if a monitored event occurs that exceeds the dynamic threshold the IMD 302 automatically reverts back to an initial state in real time. In one example the dynamic threshold can extend or replace the static persistent state threshold such that the dynamic threshold can provide additional time to complete a test before reverting IMD 302 functionality is required. In another example the dynamic threshold could depend on a state of the communication channel between the remote electronic device 304 and local programming electronic device 306. So, if a disconnect or detection of loss of connection occurs and is immediately reported, the threshold could be considered automatically reached or achieved, resulting in reverting the IMD 302 back to its previous operational state without waiting for a threshold timer (or counter) to be reached.

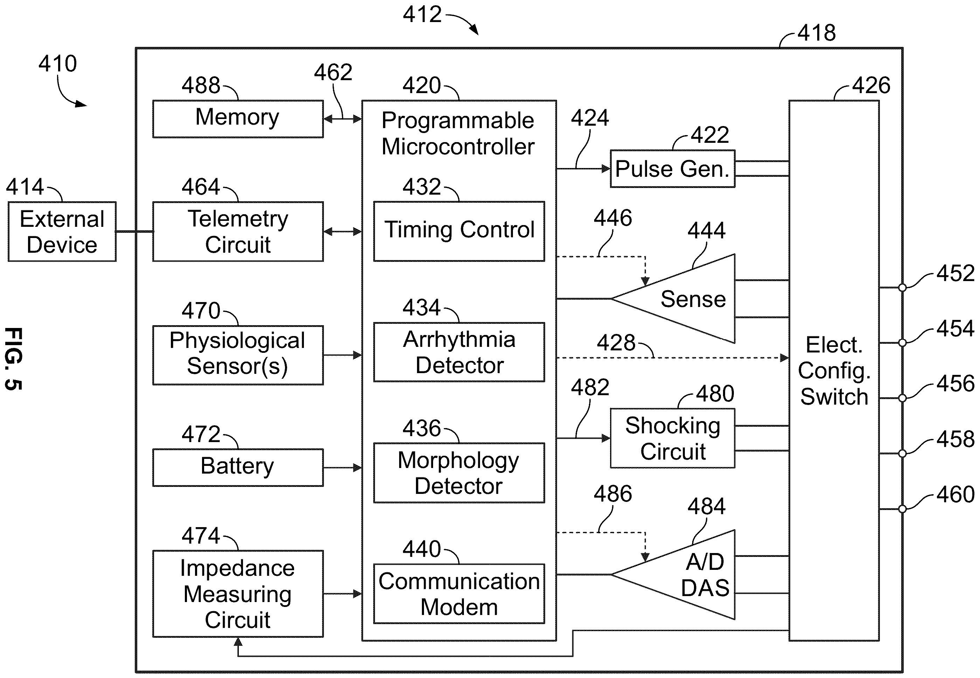

The remote electronic device 304 and the local programming electronic device 306 include respective controller circuits (e.g., controllers 316, 322) and communication circuits 318, 320. Each of the controllers includes one or more processors. In the process described above, the controller 316 of the remote electronic device 304 generates the programming package 308. The communication circuit 318 of the remote electronic device 304 communicates the programming package 308 to the local programming electronic device 306 via the network 310. The communication circuit 320 of the local programming electronic device 306 receives the programming package 308 and conveys the package 308 to the IMD 302. The communication circuit 320 may include an antenna 324 that wirelessly transmits the programming package 308 via RF signals, such as Bluetooth. The signals that represent the programming package 308 penetrate the body 326 of the patient and reach the receiver of the IMD 302. The receiver may be the communication modem 440 shown in Figure 5, which conveys the received data packets to the one or more processors of the controller 316 for analysis. In this way, the IMD 302 is beneficially able to receive the programming package 308 without requiring a network connection or a proximity sensor.

In another example, the local programming electronic device 306 processes payload 314 and updates the programming of the IMD 302 accordingly as if it had been directed by a local user. The request may be a singular input (such as a button press) or a package of inputs as you described earlier. Cybersecurity controls exist to ensure the payload 314 within the package 308 is valid in either case before the local programming electronic device 306 operates on it, and before communicating updates to IMD 302.

In instances as described above in which the communication of the programming package 308 from the source to the IMD 302 is delayed and/or indirect, there is a risk that the contents of the programming package 308 may be tampered with or corrupted after generation and before receipt by the IMD 302. The communication system 300 and method described herein may perform cryptographic operations to ensure that the configuration change requests, or payload 314 are immutable and that the source of the package 308 is authenticated prior to execution by the IMD 302.

In yet another example, a hybrid option can be provided where security credentials and characteristics apply both to the higher-level message, or package 308 as well as to the internal message, or payload 314, but only the internal message, or payload 314 is forwarded to the IMD 302. In other words, the local programming electronic device 306 forwards only the payload 314 to the IMD 302. The local programming electronic device 306 confirms the contents of the package 308 are valid before allowing payload 314 to be sent to the IMD 302. In one example the validation can be based on similar but different security characteristics (e.g., keys) used with request, payload 314 which is still validated by the IMD 302, or the controls exist when establishing the communication between the local programming electronic device 306 and the IMD 302. For example, the message of package 308 may be signed with a key known only to local programming electronic device 306. If the local programming electronic device 306 determines the package 308 to be valid, the payload 314 is extracted and processed by local programming electronic device 306. A secure connection is established with IMD 302 using suitable cyber controls (independent of this system). Once connected, the result of operations contained within message, or payload 314 result in the controller 322 within local programming electronic device 306 sending the desired configuration updates to IMD 302. Alternatively, the message of package 308 is signed with a key known only to local programming electronic device 306. If local programming electronic device 306 determines the package 308 to be valid, the payload 314 is extracted and sent to the IMD 302. The message of payload 314 is itself signed with a key known only to the IMD 302, which can then be validated as previously described.

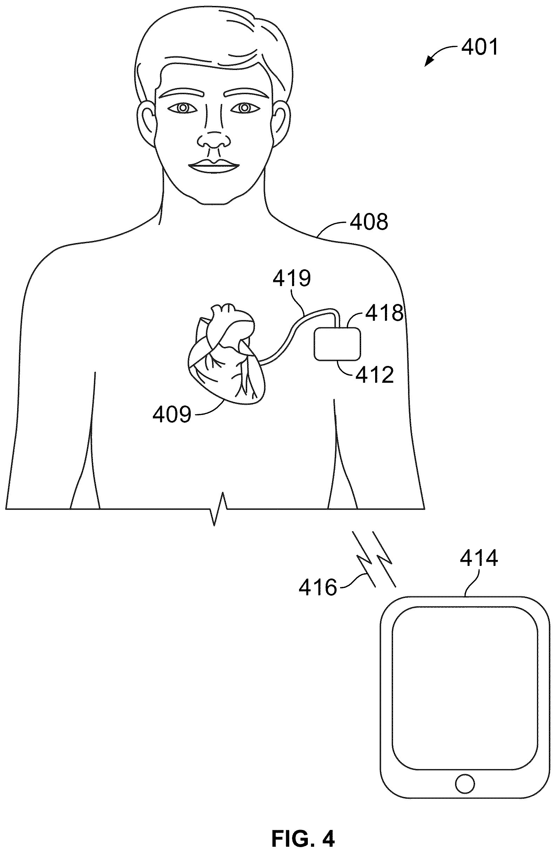

Figure 4 illustrates a system 401 that includes an IMD 412 that can couple to one or more external devices or instruments 414. A single external device 414 is illustrated in Figure 4. In example embodiments, the external device 414 can be a remote electronic device, a remote server, or the like. The IMD 412 and the external device 414 are configured to communicate with one another wirelessly over a wireless communication link 416 via a local programming electronic device.

The IMD 412 is implanted within a patient 408 at a site near the heart 409. The IMD 412 may be a cardiac pacemaker, an implantable cardiac monitoring device (ICM), a defibrillator, an ICM coupled with a pacemaker, or the like. The IMD 412 is intended for implantation within a subcutaneous pocket of the patient 408. The IMD 412 may be configured to sense cardiac signals to monitor cardiac activity over time. Optionally, the IMD 412 may also be configured to deliver stimulation therapy to the heart 409. For example, the IMD 412 may be a dual-chamber stimulation device capable of treating both fast and slow arrhythmias with stimulation therapy, including cardioversion, defibrillation, and pacing stimulation, as well as being capable of detecting heart failure, evaluating its severity, tracking the progression thereof, and controlling the delivery of therapy and warnings in response thereto.

The IMD 412 in the illustrated embodiment includes a body or housing 418 that is connected to at least one lead 419. A single lead 419 is shown in Figure 4, but the IMD 412 may include multiple leads in another embodiment. The lead 419 extends from the housing 418 to the heart 409, such that the distal end is in contact with patient tissue surrounding the heart 409. The lead 419 includes one or more electrodes that may measure cardiac signals of the heart 409 and deliver stimulation therapy to the heart 409. In an embodiment, a single electrode may emit a stimulation pulse in a stimulation mode and then may quickly switch to a monitoring mode to detect cardiac signals following the stimulation pulse. For devices which are not used for managing cardiac rhythms, the role of the heart may be replaced or represented by the appropriate anatomy, including the brain, the spine, the veinous system, the lungs, or the like.

Although the IMD 412 in the illustrated embodiment includes a lead 419, one or more of the embodiments described herein utilize a leadless IMD that does not include any lead. For example, the IMD 412 may be a leadless pacemaker, a leadless cardiac monitoring device (ICM), or the like. In general, the IMD 412 may represent a cardiac monitoring device, pacemaker, cardioverter, cardiac rhythm management device, defibrillator, neurostimulator, leadless monitoring device, leadless pacemaker, and the like. For example, the IMD 412 may include one or more structural and/or functional aspects of the device(s) described in U.S. Patent 9,333,351 "Neurostimulation Method And System To Treat Apnea"; U.S. Patent 9,044,610 "System And Methods For Providing A Distributed Virtual Stimulation Cathode For Use With An Implantable Neurostimulation System"; U.S. Patent No.: 10,765,860 titled "Subcutaneous Implantation Medical Device With Multiple Parasternal-Anterior Electrodes" and filed May 7, 2018; U.S. Pat. No.: 10,722,704, titled "Implantable Medical Systems And Methods Including Pulse Generators And Leads" filed May 7, 2018; and U.S. Pat. No.: 11,045,643 titled "Single Site Implantation Methods For Medical Devices Having Multiple Leads", filed May 7, 2018. Additionally, or alternatively, the IMD may be a leadless implantable medical device (LIMD) that include one or more structural and/or functional aspects of the device(s) described in U.S. Patent 9,216,285 "Leadless Implantable Medical Device Having Removable And Fixed Components" and U.S. Patent 8,831,747 "Leadless Neurostimulation Device And Method Including The Same". Additionally or alternatively, the IMD may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device". Additionally, or alternatively, embodiments may be implemented utilizing all or portions of the methods and systems described in U.S. Patent Application Publication Number 2021/0020294, filed 7/16/2020 and titled "METHODS, DEVICES AND SYSTEMS FOR HOLISTIC INTEGRATED HEALTHCARE PATIENT MANAGEMENT".

In another example, the IMD 412 may be an ICM that includes one or more structural and/or functional aspects of the device(s) described in U.S. Patent Publication No. 20210020294A1, filed March 29, 2016, entitled, "Method And System To Discriminate Rhythm Patterns In Cardiac Activity".

The housing 418 may contain a battery, pulse generation circuitry, communication circuitry, a data storage device (e.g., memory), and/or control circuitry including one or more processors. The control circuitry can be for receiving and analyzing biometric data such as electrocardiogram IEGM signals from electrodes, heart rate, infusion pump values, or the like. The control circuitry may include at least one processor that in this example embodiment can process the IEGM signals in accordance with algorithms to make determinations about the state of the heart 409. The memory for this embodiment provides storage for the cardiac signals and programmed instructions for the control circuitry that provide a communication session that can include a device session during which a dynamic session occurs. The battery powers the circuitry with the housing 410. For example, the battery powers the pulse generation circuitry to generate stimulation pulses and powers the communication circuitry to communicate with an external device 414. The control circuitry may generate messages to be communicated via the communication circuitry to the external device 414. The messages may include the IEGM signals and/or data generated based on the IEGM signals. The control circuitry is also configured to analyze messages, received via the communication circuit from the external device 414, that include programming packages for updating the operating configuration, including settings, parameters, behavior, and the like, of the IMD 412. Exemplary structure for the IMD 412 is discussed and illustrated below in connection with Figure 5. While illustrated as an IMD 412 that is a heart monitor, in other embodiments other medical devices can be utilized that provide other biometric data.

The external device 414 may represent a computing device that is accessible or possessable by the patient or a clinician, such as a tablet computer, a smartphone, a wearable device, laptop computer, a desktop computer, a bedside monitor installed in a patient's home, or the like. The external device 414 alternatively may be a local programming electronic device used by a clinician, nurse, technician, or the like. The external device 414 may be one of the devices listed above that is communicatively connected to a local programming electronic device that represents the source of the programming package intended to program the operating configuration of the IMD 412. For example, the local programming electronic device may be a remote server or another computing device that is communicatively connected to the remote electronic device via a network connection (e.g., the Internet). In general, the external device 414 facilitates access by physicians to patient data as well as permits the physician to review real-time electrocardiogram (ECG) signals and, as specifically described herein, program the operating configuration of the IMD 412 without the clinician being near the patient 408.

Figure 5 shows a block diagram of the IMD 412. The housing 418 or case of the IMD 412 holds the electronic and/or computing components. The housing 418 further includes a connector (not shown) with at least one terminal 452 and optionally additional terminals 454, 456, 458, 460. The terminals 452, 454, 456, 458 may be connected to electrodes that are located in various locations within and about the heart, such as on the lead 419 (shown in Figure 4). The electrodes may include various combinations of ring, tip, coil, shocking electrodes, and the like.

The IMD 412 includes a programmable microcontroller 420 that controls various operations of the IMD 412, including cardiac monitoring and stimulation therapy. Microcontroller 420 includes one or more processors (e.g., a microprocessors or equivalent control circuitry), RAM and/or ROM memory, logic and timing circuitry, state machine circuitry, and I/O circuitry. Microcontroller 420 may include an arrhythmia detector 434 that is configured to detect cardiac activity data to identify potential atrial fibrillation (AF) episodes as well as other arrhythmias (e.g., Tachycardias, Bradycardias, Asystole, etc.).

An electrode configuration switch 426 is optionally provided to allow selection of different electrode configurations under the control of the microcontroller 420. The electrode configuration switch 426 may include multiple switches for connecting the desired electrodes to the appropriate I/O circuits, thereby facilitating electrode programmability. The switch 426 is controlled by a control signal 428 from the microcontroller 420. Optionally, the switch 426 may be omitted and the I/O circuits directly connected to a housing electrode.

The IMD 412 may include a chamber pulse generator 422 that generates stimulation pulses for connecting the desired electrodes to the appropriate I/O circuits, thereby facilitating electrode programmability. The pulse generator 422 is controlled by the microcontroller 420 via control signals 424. The IMD 412 includes a sensing circuit 444 selectively coupled to one or more electrodes that perform sensing operations through the switch 426 to detect cardiac activity. The sensing circuit 444 may include dedicated sense amplifiers, multiplexed amplifiers, or shared amplifiers. The sensing circuit 444 may operate in a unipolar sensing configuration or a bipolar sensing configuration. The output of the sensing circuit 444 is connected to the microcontroller 420 which, in turn, triggers, or inhibits the pulse generator 422 in response to the absence or presence of cardiac activity. The sensing circuit 444 receives a control signal 446 from the microcontroller 420 for purposes of controlling the gain, threshold, polarization, and timing of any blocking circuitry (not shown) coupled to the sensing circuit.

The IMD 412 further includes an analog-to-digital A/D data acquisition system (DAS) 484 coupled to one or more electrodes via the switch 426 to sample cardiac signals across any pair of desired electrodes. The A/D DAS 484 is controlled by a control signal 486 from the microcontroller 420.

The IMD 412 is further equipped with a communication modem (modulator/demodulator) or circuit 440 to enable wireless communication. The modem 440 enables timely and accurate data transfer directly from the patient to the external device 414, and vice versa. For example, the communication modem 440 is configured to establish the communication link 416 with the external device 414. In an embodiment, the communication modem 440 receives a programming package from the external device 414 and conveys the programming package for updating, changing, varying, etc. the IMD 412 during a dynamic session to the microcontroller 420 for verification prior to executing configuration change requests and/or command execution requests contained within the programming package. In addition to remote programming of the IMD 412, the wireless communication link 416 with the external device 414 facilitates access by physicians and/or patients to patient data generated by the IMD 412.

The communication modem 440 may utilize radio frequency (RF), inductive, conductive, Bluetooth, or Bluetooth Low Energy (BLE) telemetry protocols. The signals are transmitted in a high frequency range and will travel through the body tissue and fluids without stimulating the heart or being felt by the patient. The communication modem 440 may be implemented in hardware as part of the microcontroller 420, or as software/firmware instructions programmed into and executed by the microcontroller 420. Alternatively, the modem 440 may reside separately from the microcontroller 420 as a standalone hardware component.

The microcontroller 420 is coupled to a non-transitory data storage device, referred to herein as memory device 488, by a suitable data/address bus 462. The memory device 488 stores programmable operating parameters used by the microcontroller 420 and/or data associated with the detection and determination of arrhythmias. In an embodiment, the memory device 488 also stores the current device session and/or dynamic session, along with any and all changes, modifications, updates, or the like previously made during a device session and/or dynamic session.

The IMD 412 optionally includes one or more physiologic sensors 470 that adjust pacing stimulation rates, detect changes in cardiac output, changes in the physiological condition of the heart, and/or diurnal changes in activity (e.g., detecting sleep and wake states). Examples of physiological sensors 470 might include sensors that, for example, sense respiration rate, pH of blood, ventricular gradient, activity, body movement, position/posture, minute ventilation (MV), and/or the like.

The battery 472 provides operating power to all of the components in the IMD 412. The battery 472 is capable of operating at low current drains for long periods of time and is capable of providing a high-current pulses (for capacitor charging) when the patient requires a shock pulse (e.g., in excess of 2 A, at voltages above 2 V, for periods of 10 seconds or more).

The IMD 412 optionally includes an impedance measuring circuit 474, which can be used for many things, including sensing respiration phase. The IMD 412 may be further equipped with a telemetry circuit 464 that can selectively communicate with an external device, such as the device 414, when connected via a physical (e.g., wired) communication link. The IMD 412 optionally includes a shocking circuit 480 controlled by control signals 482 generated by the microcontroller 420. The shocking circuit 480 generates shocking pulses of low (e.g., up to 0.5 joules), moderate (e.g., 0.5-10 joules), or high energy (e.g., 11 to 40 joules), as controlled by the microcontroller 420. In an alternative embodiment in which the IMD 412 senses and monitors cardiac activity without administering stimulation therapy, the IMD 412 may lack the pulse generator 422 and the shocking circuit 480.

The microcontroller 420 may include other dedicated circuitry and/or firmware/software components, such as a timing control (module) 432 and a morphology detector (module) 436. The timing control 432 is used to control various timing parameters, such as stimulation pulses (e.g., pacing rate, atria-ventricular (AV) delay, atrial interconduction (A-A) delay, ventricular interconduction (V-V) delay, etc.) as well as to keep track of the timing of RR-intervals, refractory periods, blanking intervals, noise detection windows, evoked response windows, alert intervals, marker channel timing, and the like. The morphology detector 436 is configured to review and analyze one or more features of the morphology of cardiac activity signals, such as the morphology of detected R waves to determine whether to include or exclude one or more beats from further analysis.

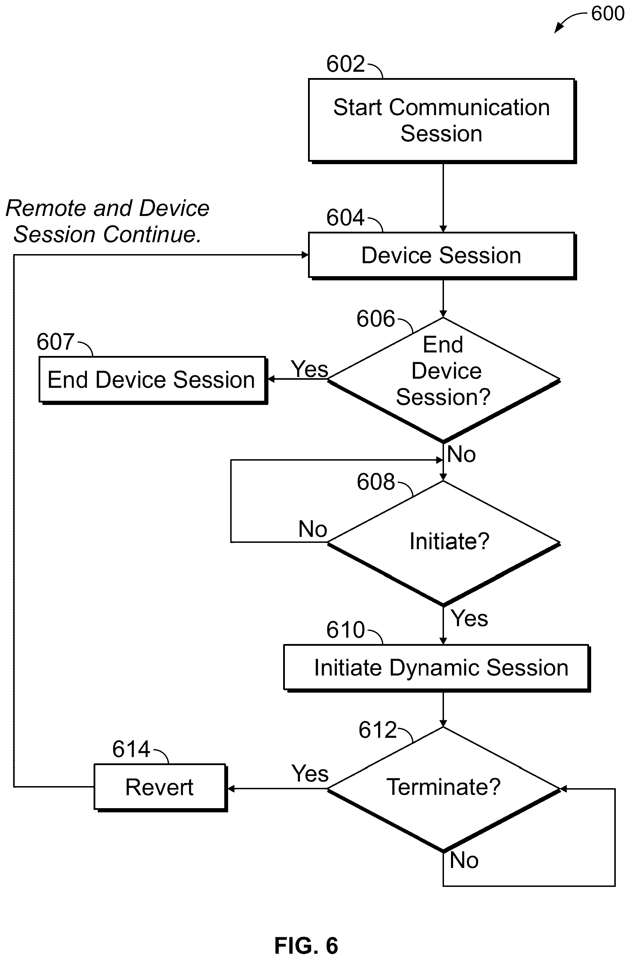

Figure 6 illustrates a method 600 for providing user input to a medical device from a remote location. In example embodiments, the systems and devices illustrated in Figures 1-5 are utilized in performing at least some if not all of the steps or functions of the method 600. To this end, when referring to one or more processors, the one or more processors can be the one or more processors of a remote electronic device remote from a patient, of a local programming electronic device local to a patient, or of a medical device including an IMD within the patient. To this end, the one or more processors can monitor, determine, and perform any action by communicating signals that cause other processors of other devices to perform the functions recited.

The method 600 presents a method of providing a dynamic session to update, change, modify, alter, interrogate or the like a medical device. The dynamic session in one example can be considered a programming session where the dynamic occurrence (e.g., update, change, modification, alteration, interrogation, etc.) to the medical device involves software, including updating, changing, modifying, altering, or the like. In addition, during such a programming session the dynamic occurrence can be to program parameters such as intervals, thresholds, or the like. In another example the dynamic session may be a period of time when the medical device is being interrogated. The dynamic session in one example may occur during a communication session between a remote electronic device and a local programming device implemented to program a medical device at the location of the local programming device. In one example the remote electronic device can be operated by a clinician, clinician assistant, programming expert, or the like. In one embodiment, the local programming electronic device can be utilized by a local clinician, such as nurse, device technician, physician, or other individual adequately trained to use and support such systems in the same room, or same environment as the patient and can be present to monitor the patient during the remote programming of the medical device. In an alternative embodiment, the remote programming device, in addition to communicating with a local electronic device, may communicate during the communication session and the dynamic session directly with the medical device without the use of a local programming electronic device. In yet another alternative embodiment, a local electronic device is provided; however, the local electronic device does not monitor the medical device or make determinations related to the communication session, the device session, or the dynamic session. Still, the local electronic device is provided in the communication pathway between the remote electronic device and the medical device.

At 602, one or more processors of a remote programming electronic device and/or local programming electronic device start a communication session between the remote programming electronic device and the local programming electronic device. In one example, the local programming electronic device can initiate the communication session, whereas in another example the remote electronic device can initiate the communication session. In one embodiment the local programming electronic device initiates the communication session, and then the local user gives control of the local programming electronic device to the user of the remote electronic device. In example embodiments, during this process security measures, such as passwords, key exchanges, randomly generated number exchanges, or the like can be implemented to ensure security over the communication session and the dynamic session that occurs during the communication session. In example embodiments a third-party electronic device may provide the key, random number, etc. to initiate the communication session. As a result of the communication session, the remote user of the remote electronic device is able to control the local programming electronic device. In this manner, an input provided at the remote electronic device can be communicated to the local programming electronic device and executed by the local programming electronic device.

At 604, the one or more processors of the local programming electronic device begin a device session with a local medical device. In one example the medical device is an IMD. During the initiation of the device session between the local programming electronic device and the medical device, the remote electronic device remains in communication and in control of the local programming electronic device and is, thus, in control of the device session with the medical device. Again, security measures, including key exchanges, passwords, etc. can be utilized during the initiation of the device session. In an alternative embodiment, the one or more processors of the remote electronic device begin the device session directly with the medical device. In particular, in an example embodiment the local programming electronic device may be eliminated such that only a transceiver within the medical device communicates with the remote electronic device. In yet another example the local programming electronic device may simply be a local electronic device only used as a communication conduit between the remote electronic device and the medical device. In this manner, the communication session can include the remote electronic device and the local electronic device, or the remote electronic device, local electronic device, and medical device.

At 606, the one or more processors of the remote electronic device, the local programming electronic device, and/or the medical device make determinations whether to terminate the device session. During the device session and before beginning the dynamic session, any device in the communication session (e.g., the remote electronic device, the local programming device, and the medical device) can cause termination of the device session. In one example a manual input at the remote electronic device or the local programming electronic device can result in the termination. So, if a remote user is not satisfied with a programming update that is to occur, if a local user recognizes a patient is not in a condition to receive the programming update, or the like, the device session can be terminated. Similarly, if a connectivity issue occurs over the network, an electronic device runs out of battery, the communication session is accidentally terminated by a user, or the like occurs, again, the device session may be automatically terminated.

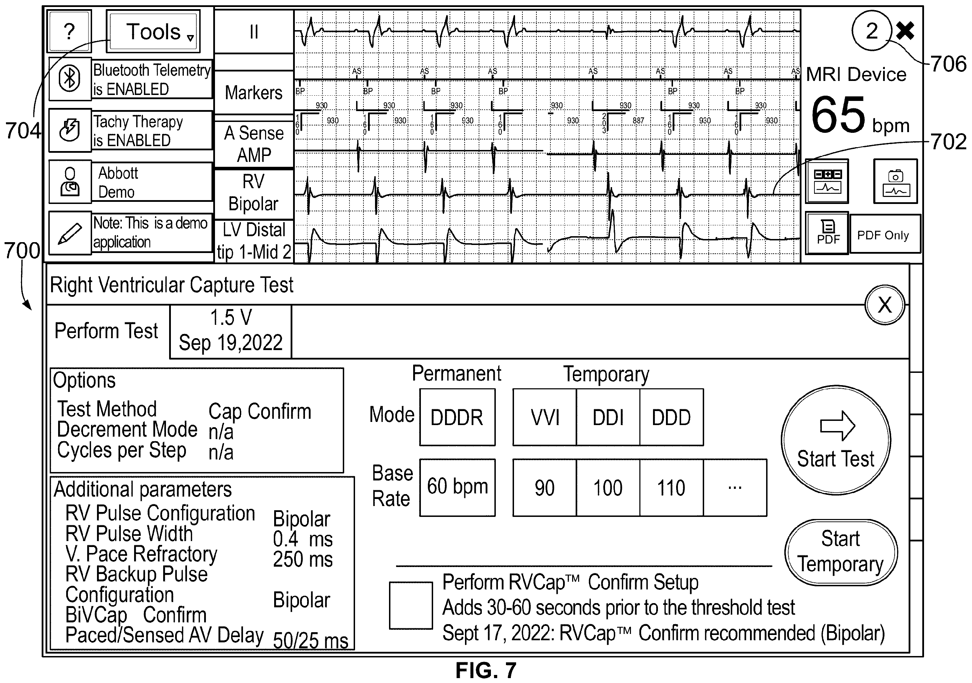

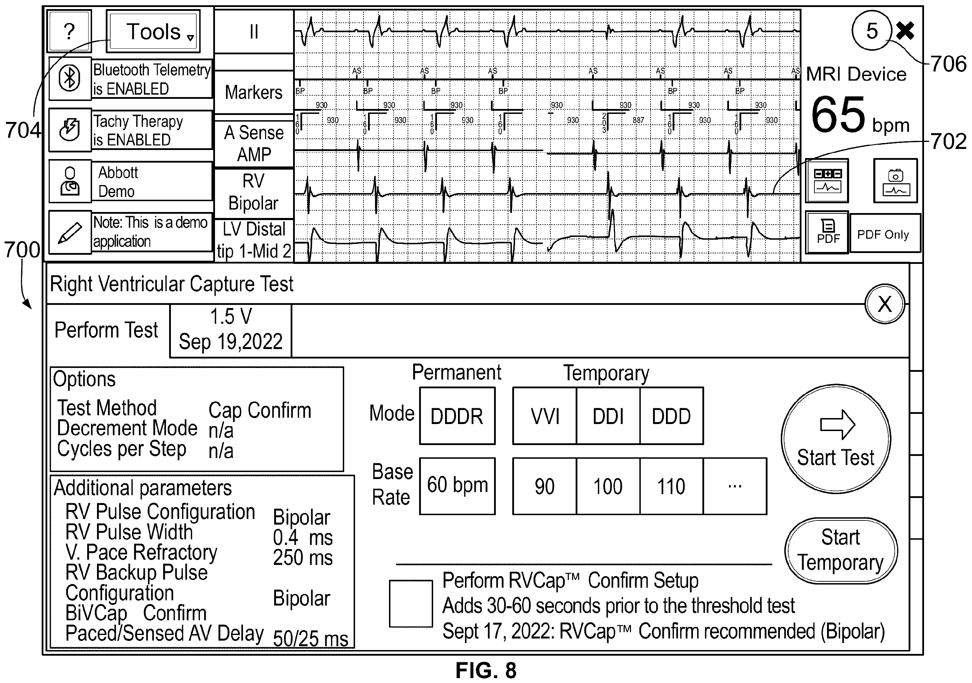

If at 606 the device session is not terminated, then at 608, a determination can be made whether a dynamic session is ready to be initiated during the device session. For example, a user of the remote electronic device can determine if all programming updates/changes are present, or if additional programming updates/changes need to be downloaded or obtained. In another example thresholds related to the dynamic session can be set, modified, changed, updated, or the like. For example, a persistent state threshold can be set, modified, changed, updated, or the like. Figures 7 and 8 illustrate example input screens 700 related to the programming, changing, varying, modifying, of a medical device that can be observed during the device session before starting a dynamic session. As illustrated, the input screen can include patient health information 702, such as heart related signals, heart information such as beats per minute, or the like. The input screen 700 can also include input buttons 704 to allow a user to navigate the programming and have functionality over the communication session, device session, dynamic session, and information related to the patient and each session. In addition, the input screen may include a persistent state threshold input 706. The persistent state threshold input 706 represents the specified period of time a persistent state is allowed to continue during the dynamic session before the local programming electronic device terminates the dynamic session with the medical device, thus reverting the medical device and/or the local programming electronic device to their pre-programming state.

The persistent state threshold input 706 can start as a default amount, such as 2 seconds (Figure 7), and then can be changed or varied to a second determined amount, such as 5 seconds (Figure 8), for a particular dynamic session. In one example, the persistent state threshold input 706 may be manually changed by the clinician at the remote electronic device via an input button or key. The change can be based on a preference of the clinician, a determination based on the programming being conducted or the like.

Alternatively, the one or more processors can automatically change the persistent state threshold based on patient characteristics, clinician characteristics, medical device characteristics, monitoring characteristics, etc. For example, clinician characteristics may be utilized. The clinician using the remote electronic device may be identified by the one or more processors as a result of a login, sensor information (e.g., camera information), or the like (e.g., clinician characteristics), and the change is based on a preference of the identified clinician. To this end, the clinician may have a profile such that any time that particular clinician is the user of the remote electronic device the persistent state threshold input is automatically set to the desired value of that clinician.

In another example, patient characteristics may be utilized to determine the persistent state threshold. Patient characteristics can include age, health status, health conditions, weight, previous health events, or the like (e.g., risks). These patient characteristics can be utilized with an algorithm, model, mathematical function, artificial intelligence, lookup table, decision tree, or the like to determine the persistent state threshold based on risk to the patient.

In yet another example, the one or more processors can determine the persistent state threshold based on medical device characteristics. The medical device characteristics can be determined based on the type of medical device, age, brand, status, battery capacity, the programming update itself, or the like.

In another example, monitoring characteristics can be utilized to determine the persistent state threshold. Monitoring characteristics include any characteristics that can be obtained using a sensor of the local programming electronic device or the medical device. Example monitoring characteristics include connectivity status, communication signal strength, measures related to current electrical environment, electrical noise level, or the like. In addition, such monitoring characteristics can include sensor information related to the patient, including patient characteristics. To this end, some characteristics can be considered more than one type of characteristic. Additional sensor information can include characteristics related to the local user, such as camera or image characteristics. In particular, if the local user leaves the environment where the communication session is taking place, the patient's health changes, or the like, such image data is considered a monitoring characteristic.

Regardless of the type of characteristic obtained, based on the characteristic(s), a determination can be made regarding the persistent states that may occur during the dynamic session and a predicted amount of time each persistent state should last. In yet other examples the persistent state threshold can be determined utilizing a combination of these characteristics and the methodologies provided.

With reference back to the method 600 of Figure 6, if at 608 the dynamic session is not ready to initiate, then the device session continues allowing additional changes and modifications prior to the start of the dynamic session. In particular, there is a desire to have all programs, changes, thresholds, settings, etc. in place and set prior to beginning the dynamic session. In this manner, once the dynamic session begins, the minimum amount of time possible can be spent during the dynamic session. Such result reduces risk and enhances safety for the patient.

If at 608 the dynamic session is ready to begin, then at 610 the one or more processors of the local programming electronic device initiate the dynamic session. During the dynamic session the remote electronic device user can provide inputs via the local programming electronic device to modify, update, change, etc. one or more programs of the medical device, run tests on the medical device, or the like. Such modifications, updates, changes, etc. can alter the settings or programmed configuration of the medical device or alternatively can include adding a new program, and/or deleting an old program. These modifications, updates, changes, or the like can result from an extended persistent action by the remote user, including holding down input keys or input devices for a determined period of time or until the set of tests, modifications and/or updates of the initiating action have completed.

At 612, the one or more processors of the local programming electronic device determine whether to terminate the dynamic session. In one example, if a persistent action exceeds a persistent state threshold, the dynamic session can be automatically terminated. In this manner, if a connectivity issue, system error, or the like occurs that can negatively affect the dynamic session, and hence the patient, the dynamic session can be automatically terminated, and at 614 the one or more processors of the local programming electronic device can revert the medical device back to its previous programming. If the local user detects a change in the patient's condition, or the remote user observes any other unexpected software or system or patient response (i.e. a stuck input key) the local user can manually terminate the dynamic session, whereby at 614 the one or more processors of the local programming electronic device revert the medical device back to its previous programming. This reduces the risk to the patient and enhances patient safety. Additionally, the termination of the dynamic session does not automatically terminate the communication session between the remote electronic device and local programming electronic device, or the device session between the local programming electronic device and the medical device. As a result, if the issue that resulted in the termination of the dynamic session can be resolved (e.g., connectivity returns, remote user unsticks input key, etc.) another attempt at a dynamic session can be immediately undertaken. Thus, patient safety is achieved with only a slight interruption of the updating, modifying, changing, etc. of the medical device.

In addition, at 612, during the dynamic session dynamic changes associated with the dynamic session can be monitored to determine whether to terminate the dynamic session for a different reason other than a persistent action. For example, the patient may be continuously and repeatedly monitored for health characteristics to ensure that the functionality of the medical device is not needed during the dynamic session. The health characteristics can include changes in heartbeats per minute, changes in cardiac signals, changes in consciousness of the patient, changes in temperature, or the like that may indicate that the patient may need immediate use of the medical device. Other dynamic changes can include those input by the user of the local programming electronic device. This user (e.g., nurse, technician, or the like) is observing the patient during the dynamic session and can provide an input that the dynamic session should be terminated. In yet another example, the internet connection can be monitored to determine if or when a communication connection has been lost.

In addition, at 610 or 612, after initiating the remote programming update, the remote or local user or the system may terminate the communication session at any time. Doing so results in detection of the loss of communication by the local programming electronic device upon reaching the persistent state threshold resulting in termination. At 614, in response to termination the local programming electronic device reverts the programming, or state, of the medical device to its original state before the start of the dynamic session. Then the process returns to step 604 where the device session remains active, but the communication system is not active thereby preventing further remote programming actions and ending this method 600.

The dynamic monitoring may be used independently or to extend the utility of static thresholds. Dynamic monitoring would end the persistent action only upon detecting a change in the state of the system (such as due to the loss of communication between the remote electronic device and the local programming electronic device, a change in state of the local programming electronic device or of a connected system such as an implantable device, a user input from the local user at the local programming electronic device, a change in state or condition of a patient or the patient's implantable device as detected through any channel such as an external heart monitor or alarm, etc.). If the change in state cannot be detected directly, and instantaneously, use of the fixed static persistent state threshold would expect to be used in addition to the periodic monitoring of the dynamic input(s). For example, the beginning of the static time evaluation is reverted periodically each time the dynamic status is successfully re-evaluated. Thus, the persistent action is automatically terminated only after the combination of reaching the pre-determined static persistent state threshold since the last dynamic status event was re-evaluated.

Though some dynamic status events may be independently monitored completely within the local programming electronic device, other status events may involve the periodic transfer of information between devices. For example, the remote electronic device (or other externally monitored source of input) may periodically (such as once every 100 ms, once per second, once per minute, etc.) send a status update to the local programming electronic device to confirm communication between the two devices is present, that communication latencies are adequate for such actions, or that some other criteria essential to safety or performance of the system remains satisfied. If the static persistent state threshold, described above, is reached after the most recent periodic status update is received by the local programming electronic device, this would be interpreted as a loss of communication necessitating the automatic end of the persistent action, because any input from the remote electronic device could not expect to be transmitted to the local programming electronic device when that communication channel is not available. Thus at 612, all sources of information described previously would be used to evaluate the combination of all available dynamic inputs and manual user inputs collected during persistent actions, within the currently defined static persistent state threshold, which then transitions to 614 and terminates the programming state due to an exception from any monitored criteria described above.

In a first example of the method 600 where a dynamic status monitored interruption control with a static persistent state threshold is provided. A periodic status message is transmitted (i.e., every 0.5 to 1 second) from the remote electronic device to the local programming electronic device. Optionally, this could be limited to the periods of time where a persistent (remote) user input is active or could be continuous when a remote electronic device connection exists with the local programming electronic device. Each time this status message is received, a static timer or counter on the local programming electronic device is restarted. If no status message is received before the static timer expires, the local programming electronic device signals a loss in communication with the remote electronic device to any software processes that subscribe to such status update events.

In the case where a remote user at the remote electronic device starts a persistent action which temporarily changes the state of the system (i.e. patient's device), and communication with the remote electronic device is lost while that persistent action is still in progress, the static timer expires after the defined persistent state threshold since receipt of the last status message. This automatically terminates the persistent user action (button release or otherwise) which triggers the local programming electronic device to restore the operation of the IMD from its temporary state. Similarly, the system may allow for two or more attempts to evaluate the conditions before terminating the persistent user action. This is to allow for cases where the monitoring interval (i.e., the rate such characteristics are updated) is short enough to support two or more samples to be collected within the static or dynamic thresholds established for the action being performed. In one example, three samples are collected.

In a second example, a remotely configurable static persistent state threshold may be provided. A remote input (e.g., input button) of the remote electronic device may be utilized to manually set the persistent state threshold. The shape and size of the remote input, and text fonts can be suited to match the appropriate user interface style recommendations so long as to allow the display of the currently selected static threshold value. This value could be of any suitable range and unit deemed appropriate to the applications supported by the system. In the example of Figures 7 and 8, the value (e.g., 706) is reflected as 2 seconds in Figure 7 and 5 seconds in Figure 8.

The system defaults the value (e.g., 706) to its lowest, safest, value in the supported range upon initialization or upon the selection of a new test or action. In this example, 2 seconds would be the default. In one example, upon the user pressing the remote user input button, the safety threshold value cycles through a pre-defined set of values. In this example, the next available value is 5 seconds, so upon the first press of the remote input button, the value of 5 is displayed to the user. Additional presses of the button would cycle through each available value until returning back to the initial value of the series, in this case 2 seconds. For example, a series of presses could cycle from 2 seconds to 5 seconds, to 10 seconds, to 15 seconds, to 30 seconds, to 60 seconds and then back to 2 seconds to repeat from the beginning. Any sequence could be defined. Alternatively, the user could use the keypad of the remote electronic device to input an exact number, such as 42 to set the persistent state threshold. Alternatively, the software may define the minimum, maximum or recommended selection of values which can be selected by the remote user for any given test or set of characteristics as defined previously.

In the example, the system could additionally restore the default value back to the initial value of 2 seconds due to any combination of other defined actions which may include but is not limited to the following possible examples: a) upon completion of the next Press and Hold Action (The user selectable value is only used once. The operator could be required to update the selection for each press and hold action.); b) no user interaction for an extended period of time; c) the remote user pauses/resumes the remote session; d) change in connectivity status or method detected; e) etc.

The one or more processors of the local programming electronic device can be responsible for displaying the currently selected safety threshold software to the remote user via the remote user action button. This ensures the remote user is aware of the persistent state threshold before starting a persistent action.

When the local programming electronic device is notified of a remote user action to start a new persistent action, a timer is started in the local programming electronic device using the selected persistent state threshold. If an action to end the persistent event (such as releasing a mouse button or touch screen) is not received by the local programming electronic device within this defined time limit, the local programming electronic device automatically terminates the action in real time (and any resulting restoration of the system state) as required when ending such actions. In the typical case, ending the ongoing action by releasing the button restores any temporary modifications made to an implantable devices temporary operation and restores control of the local programming electronic device back to the local user and/or remote user.

In a third example a local user can terminate a persistent action. In this example, the remote user starts a persistent action through the remote electronic device. At any time after that persistent action is started, any input made by the local user to the local programming electronic device terminates that persistent action and returns control to the local user.

In a fourth example, a dynamic status monitored interruption control for local or remote input may be provided. In this example, a patient is hooked up, or connected, to an external monitor or measuring apparatus supported by the local programming electronic device that can monitor any number of physiological properties against pre-defined thresholds including but not limited to heart rate, blood pressure, cardiac output, blood oxygen content, respiratory rate, etc. Periodically, the status of these properties is assessed by the local programming electronic device. Optionally, this assessment could be limited to the periods of time where a persistent user input is active. Each time this status is evaluated and is within the desired range, the static timer on the local programming electronic device is restarted. If the status indicates a failure, such as exceeding a programmed threshold, or if no status measurement can be obtained before the static timer expires, the local programming electronic device generates a signal(s) to any software processes that subscribe to such status update events. This automatically in real time terminates the persistent user action (button release or otherwise) which triggers the local programming electronic device to restore the operation of the system from its temporary state. Optionally, such changes in the state of one or more monitored properties could be used to block certain subsequent actions until the condition of the system or patient is restored to an acceptable level.

In a fifth example, a remote electronic device connects directly with a medical device that is local to the patient. In one example the medical device is a monitor external to the patient, whereas alternatively the medical device is an IMD. After safety protocols are met, a communication session is initiated between the remote electronic device and the medical device. At this time, because a local programming electronic device is not provided, the communication session and device session are both initiated simultaneously. During the communication session/device session, a static persistent state threshold can be set for a test to be conducted on the medical device that involves compressing a mouse button at the remote electronic device for five seconds. In such an example, the static persistent state threshold can be set at fifteen seconds such that the medical device detects the mouse button at the remote electronic device has been compressed longer than fifteen seconds, the dynamic session automatically terminates, and the medical device reverts back to its previous programming from before the initiation of the dynamic session. If any dynamic changes occur during the dynamic session, the one or more processors can terminate the dynamic session such that the medical device reverts back to its pre-dynamic session state at 614. Thus, a new attempt can be undertaken to update, modify, change, or the like the medical device. To this end, once the update/modification/change is complete, the dynamic session ends. At this time the device session and communication session can both end as well because the update/modification/change has been successfully achieved.

In a sixth example, a remote electronic device couples to the medical device via a local electronic device that does not make determinations related to the medical device or dynamic session and/or monitor the persistent actions during the dynamic session. In such an embodiment the remote user of the remote electronic device can set the persistent state threshold, and the medical device can monitor dynamic changes to the medical device, patient, dynamic session, or the like. Based on this monitoring one or more processors of the medical device may vary dynamic thresholds, or take any action as previously described in relation to the local programming electronic device.

In an example, a communication system 300 is provided. In this example, the communication system 300 comprises a local programming electronic device 306 and a remote electronic device 304. The remote electronic device 304 is configured to communicate with a medical device 302 of a patient via the local programming electronic device 306. The remote electronic device 304 including one or more processors 316 configured to control operations of the local programming electronic device 306 to program the medical device 302 during a dynamic session, and terminate the dynamic session in response to 1) a persistent action of a user of the remote electronic device 304 exceeding a static persistent state threshold and/or 2) a monitored event exceeding a dynamic threshold.

In another example, a communication system 300 is provided. In this example, the communication system 300 comprises a local programming electronic device 306 and a remote electronic device 304. The remote electronic device 304 includes one or more processors 316 configured to communicate with the local programming electronic device 306 and control the local programming electronic device 306 to program a medical device 302 of a patient. The local programming electronic device 306 includes one or more processors 322 configured to program the medical device 302 during a dynamic session and monitor persistent actions of the remote electronic device 304 during the dynamic session. The one or more processors 322 of the local programming electronic device 302 are also configured to monitor at least one of patient characteristics, medical device characteristics, or monitoring characteristics during the dynamic session, determine a dynamic threshold based on the patient characteristics, the medical device characteristics, and/or the monitoring characteristics. The one or more processors 322 of the local programming electronic device 302 are further configured to determine whether the dynamic threshold is exceeded during the dynamic session and determine whether a persistent action of the persistent actions exceeds a static persistent state threshold during the dynamic session. The one or more processors 322 of the local programming electronic device 302 are additionally configured to terminate the dynamic session when either 1) the dynamic threshold is exceeded, or 2) when the persistent action exceeds the static persistent state threshold.

### Closing

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a dynamic random-access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for conducting operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for conducting the operations and provide this code through its network interface via a network to a second device having a second processor for execution of the code on the second device.

Aspects are described herein with reference to the figures, which illustrate example methods, devices, and program products according to various example embodiments. The program instructions may be provided to a processor of a general-purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally, or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being conducted in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. A communication system (100, 300) comprising:
a remote electronic device (116, 304) configured to communicate with a medical device (114, 212, 302) of a patient via a local programming electronic device (106, 306);
the remote electronic device (116, 304) including one or more processors (202, 316) configured to:
control operations of the local programming electronic device (106, 306) to program the medical device (114, 212, 302) during a dynamic session; and
terminate the dynamic session in response to 1) a persistent action of a user (112) of the remote electronic device (116, 304) exceeding a static persistent state threshold and 2) a monitored event exceeding a dynamic threshold.

2. The communication system of claim 1, wherein the one or more processors (202, 316) are further configured to:
provide a timer (222) during the dynamic session; and
determine whether a duration of the persistent action as determined by the timer (222) exceeds the static persistent state threshold.

3. The communication system of claim 2, wherein the one or more processors (202, 316) are further configured to:
adjust the timer (222) based on a manual input from the user (112) of the remote electronic device (116, 304).

4. The communication system of claim 2 or 3, wherein the one or more processors (202, 316) are further configured to:
obtain patient characteristics, clinician characteristics, medical device characteristics, or monitoring characteristics; and
determine the static persistent state threshold based on the patient characteristics, the clinician characteristics, the medical device characteristics, or the monitoring characteristics obtained; and
update the static persistent state threshold based on the determining.

5. The communication system of any one of claims 1 to 4, wherein the one or more processors (202, 316) are further configured to:
obtain patient characteristics, medical device characteristics, or monitoring characteristics; and
determine the dynamic threshold based on the patient characteristics, the medical device characteristics, or the monitoring characteristics obtained.

6. The communication system of any one of claims 1 to 5, wherein the one or more processors (202, 316) are further configured to:
revert the medical device (114, 212, 302) to a previous configuration via the local programming electronic device (106, 306) in response to termination of the dynamic session.

7. A communication system (100, 300) comprising:
a remote electronic device (116, 304) including one or more processors (202, 316) configured to:
communicate with a local programming electronic device (106, 306); and
control the local programming electronic device (106, 306) to program a medical device (114, 212, 302) of a patient;
the local programming electronic device (106, 306) including one or more processors (202, 322) configured to:
program the medical device (114, 212, 302) during a dynamic session;
monitor persistent actions of the remote electronic device (116, 304) during the dynamic session;
monitor at least one of patient characteristics, medical device characteristics, or monitoring characteristics during the dynamic session;
determine a dynamic threshold based on the patient characteristics, the medical device characteristics, or the monitoring characteristics;
determine whether the dynamic threshold is exceeded during the dynamic session;
determine whether a persistent action of the persistent actions exceeds a static persistent state threshold during the dynamic session; and
terminate the dynamic session when either 1) the dynamic threshold is exceeded, or 2) when the persistent action exceeds the static persistent state threshold.

8. The communication system of claim 7, wherein the one or more processors (202, 322) of the local programming electronic device (106, 306) are further configured to:
provide a timer (222) during the dynamic session; and
determine whether a duration of the persistent action as determined by the timer (222) exceeds the static persistent state threshold.

9. The communication system of claim 8, wherein the one or more processors (202, 322) of the local programming electronic device (106, 306) are further configured to:
determine the static persistent state threshold based on clinician characteristics, the patient characteristics, the medical device characteristics, or the monitoring characteristics obtained; and
update the static persistent state threshold based on the determining.

10. The communication system of any one of claims 7 to 9, wherein the one or more processors (202, 322) of the local programming electronic device (106, 306) are further configured to:
revert the medical device (114, 212, 302) to a previous configuration in response to termination of the dynamic session so that the medical device (114, 212, 302) can provide a treatment to the patient.

11. The communication system of any one of claims 4, 5, 7 to 10, wherein the monitoring characteristic is related to connectivity between the remote electronic device and the local programming electronic device.

12. The communication system of any one of claims 1 to 11, wherein the dynamic session is a programming session.

13. A communication method comprising:
controlling a local programming electronic device (106, 306) with a remote electronic device (116, 304) to program a medical device (114, 212, 302) of a patient during a dynamic session;
monitoring persistent actions of the remote electronic device (116, 304) during the dynamic session;
determining whether a persistent action of the persistent actions exceeds a static persistent state threshold during the dynamic session;
terminating the dynamic session when the persistent action exceeds the static persistent state threshold; and
reverting the medical device (114, 212, 302) to a previous configuration in response to terminating the dynamic session.

14. The method of claim 13, further comprising:
monitoring at least one of patient characteristics, medical device characteristics, or monitoring characteristics during the dynamic session;
determining a dynamic threshold based on the patient characteristics, the medical device characteristics, or the monitoring characteristics;
determining whether the dynamic threshold is exceeded during the dynamic session; and
terminating the dynamic session when the dynamic threshold is exceeded.

15. The method of claim 13 or 14, wherein determining the static persistent state threshold includes at least one of 1) receiving a manual input from a remote user (112) of the remote electronic device (116, 304) or 2) determining the static persistent state threshold based on patient characteristics, clinician characteristics; medical device characteristics, or monitoring characteristics.
